(19) Europäisches Patentamt
European Patent Office
Office européen des brevets

(11) **EP 3 208 859 B1**

(12) **EUROPEAN PATENT SPECIFICATION**

(45) Date of publication and mention of the grant of the patent:
**22.05.2019 Bulletin 2019/21**

(51) Int Cl.:
*H01L 51/44* (2006.01)       *H01L 51/00* (2006.01)
*C07C 211/04* (2006.01)     *C07F 7/24* (2006.01)
*C08L 33/00* (2006.01)

(21) Application number: **15850889.5**

(22) Date of filing: **14.10.2015**

(86) International application number:
**PCT/JP2015/079095**

(87) International publication number:
**WO 2016/060185 (21.04.2016 Gazette 2016/16)**

(54) **SOLAR CELL**

SOLARZELLE

CELLULE SOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2014   JP 2014210195**

(43) Date of publication of application:
**23.08.2017   Bulletin 2017/34**

(73) Proprietor: **Sekisui Chemical Co., Ltd.
Osaka-shi
Osaka 530-0047 (JP)**

(72) Inventors:
• **HAYAKAWA Akinobu
Mishima-gun
Osaka 618-0021 (JP)**
• **ASANO Motohiko
Mishima-gun
Osaka 618-0021 (JP)**
• **UNO Tomohito
Mishima-gun
Osaka 618-0021 (JP)**
• **HORIKI Mayumi
Mishima-gun
Osaka 618-0021 (JP)**
• **FUKUMOTO Yuuichirou
Mishima-gun
Osaka 618-0021 (JP)**
• **KUREBAYASHI Tetsuya
Mishima-gun
Osaka 618-0021 (JP)**
• **OHARA Shunji
Mishima-gun
Osaka 618-0021 (JP)**

(74) Representative: **Hart-Davis, Jason et al
Cabinet Beau de Loménie
158, rue de l'Université
75340 Paris Cedex 07 (FR)**

(56) References cited:
EP-A2- 2 056 372          WO-A1-2013/171517
WO-A1-2014/097299     WO-A2-2014/042449
JP-A- 2002 198 539       JP-A- 2005 154 528
JP-A- 2014 056 962

EP 3 208 859 B1

## Description

TECHNICAL FIELD

The present invention relates to a solar cell that is excellent in photoelectric conversion efficiency, suffers little degradation during encapsulation (initial degradation), has high-temperature durability, and is excellent in temperature cycle resistance.

BACKGROUND ART

Photoelectric conversion elements equipped with a laminate having an N-type semiconductor layer and a P-type semiconductor layer disposed between opposing electrodes have been conventionally developed. Such photoelectric conversion elements generate photocarriers by photoexcitation so that electrons and holes move through the N-type semiconductor and the P-type semiconductor, respectively, to create an electric field.

Most photoelectric conversion elements currently in practical use are inorganic solar cells which are produced using inorganic semiconductors made of silicon or the like. The inorganic solar cells, however, are utilized only in a limited range because their production is costly and upsizing thereof is difficult. Therefore, organic solar cells produced using organic semiconductors instead of inorganic semiconductors have received attention.

In organic solar cells, fullerene is used in most cases. Fullerene is known to function mainly as an N-type semiconductor. For example, Patent Literature 1 discloses a semiconductor heterojunction film formed using an organic compound serving as a P-type semiconductor, and fullerenes. Fullerene, however, is known to be responsible for degradation of organic solar cells produced using the fullerene (see e.g., Non-Patent Literature 1). Thus, there is a demand for a material that substitutes for fullerene.

In the organic solar cells, a laminate having an N-type semiconductor layer and a P-type semiconductor layer disposed between opposing electrodes is generally encapsulated using an encapsulation resin such as a sealing material (see e.g., Non-Patent Literature 2). However, the problem of the organic solar cells encapsulated using an encapsulation resin such as a sealing material is that, depending on the type of a semiconductor material, the semiconductor material is degraded during encapsulation, resulting in reduced photoelectric conversion efficiency (initial degradation).

CITATION LIST

- Patent Literature

Patent Literature 1: JP 2006-344794 A

- Non-Patent Literature

Non-Patent Literature 1: Reese et al., Adv. Funct. Mater., 20, 3476-3483 (2010)
Non-Patent Literature 2: Proc. of SPIE Vol. 7416 74160K-1

An organic device of EP 2 056 372 A2 has a gas barrier film having a gas barrier layer and an antistatic layer containing an acrylic or methacrylic resin.

WO 2014/097299 A1 shows a solar cell according to the preamble of claim 1.

SUMMARY OF INVENTION

- Technical Problem

An object of the present invention is to provide a solar cell that is excellent in photoelectric conversion efficiency, suffers little degradation during encapsulation (initial degradation), has high-temperature durability, and is excellent in temperature cycle resistance.

- Solution to Problem

The present invention provides the solar cell of claim 1 including: a laminate having an electrode, a counter electrode, and a photoelectric conversion layer disposed between the electrode and the counter electrode; and an encapsulation material covering the counter electrode to encapsulate the laminate, the photoelectric conversion layer including an organic-inorganic perovskite compound represented by the formula R-M-X$_3$, R representing an organic molecule, M representing a metal atom, X representing a halogen atom or a chalcogen atom, the encapsulation material including a (meth)acrylic resin having a C atom/O atom ratio of 4 or more in the molecule.

Hereinafter, the present invention will be described in detail.

The present inventor studied use of a particular organic-inorganic perovskite compound for a photoelectric conversion layer in a solar cell in which a laminate having an electrode, a counter electrode, and a photoelectric conversion layer disposed between the electrode and the counter electrode is encapsulated with an encapsulation material. Use of the organic-inorganic perovskite compound can be expected to improve the photoelectric conversion efficiency of the solar cell.

However, encapsulation of a laminate including a photoelectric conversion layer containing the organic-inorganic perovskite compound with a conventional encapsulation material was found to reduce the photoelectric conversion efficiency during the encapsulation (initial degradation).

The present inventors conducted intensive studies on the cause of degradation that occurs when a laminate including a photoelectric conversion layer using an organic-inorganic perovskite compound is encapsulated with an encapsulation material. The present inventors consequently found that this problem arises because, during encapsulation, an organic component in the organic-inorganic perovskite compound is dissolved into the encapsulation material so that the organic-inorganic perovskite compound is degraded.

The present inventors conducted diligent studies to consequently find that use of a (meth)acrylic resin having a C atom/O atom ratio of 4 or more in the molecule as the encapsulation material can prevent an organic component in the organic-inorganic perovskite compound from being eluted during encapsulation. The present inventor further found that use of a particular (meth)acrylic resin having relatively high hydrophobicity as the encapsulation material can also improve the high-temperature durability and temperature cycle resistance of the resulting solar cell. On the basis of these findings, the present invention has been completed.

The solar cell of the present invention includes: a laminate having an electrode, a counter electrode, and a photoelectric conversion layer disposed between the electrode and the counter electrode; and an encapsulation material covering the counter electrode to encapsulate the laminate.

The term "layer" as used herein means not only a layer having a clear boundary, but even a layer having a concentration gradient in which contained elements are gradually changed. The elemental analysis of the layer can be conducted, for example, by FE-TEM/EDS analysis and measurement of the cross section of the solar cell to confirm the element distribution of a particular element. The term "layer" as used herein means not only a flat thin film-shaped layer, but also a layer capable of forming an intricate structure together with other layer(s).

The materials of the electrode and the counter electrode are not particularly limited, and conventionally known materials may be used. The counter electrode is often a patterned electrode.

Examples of the materials of the electrode and the counter electrode include fluorine-doped tin oxide (FTO), sodium, sodium-potassium alloys, lithium, magnesium, aluminum, magnesium-silver mixtures, magnesium-indium mixtures, aluminum-lithium alloys, Al/Al$_2$O$_3$ mixtures, Al/LiF mixtures, metals such as gold, CuI, conductive transparent materials such as indium tin oxide (ITO), SnO$_2$, aluminum zinc oxide (AZO), indium zinc oxide (IZO) and gallium zinc oxide (GZO), and conductive transparent polymers. These materials may be used alone or may be used in combination of two or more thereof.

The electrode and the counter electrode may each be either a cathode or an anode.

The photoelectric conversion layer includes an organic-inorganic perovskite compound represented by the formula R-M-X$_3$ wherein R represents an organic molecule, M represents a metal atom, and X represents a halogen atom or a chalcogen atom.

Use of the organic-inorganic perovskite compound in the photoelectric conversion layer can improve the photoelectric conversion efficiency of the solar cell.

The R is an organic molecule and is preferably represented by C$_l$N$_m$H$_n$ (1, m and n each represent a positive integer).

Specific examples of the R include methylamine, ethylamine, propylamine, butylamine, pentylamine, hexylamine, dimethylamine, diethylamine, dipropylamine, dibutylamine, dipentylamine, dihexylamine, trimethylamine, triethylamine, tripropylamine, tributylamine, tripentylamine, trihexylamine, ethylmethylamine, methylpropylamine, butylmethylamine, methylpentylamine, hexylmethylamine, ethylpropylamine, ethylbutylamine, formamidine, guanidine, imidazole, azole, pyrrole, aziridine, azirine, azetidine, azete, azole, imidazoline, carbazole and their ions (e.g., methylammonium (CH$_3$NH$_3$)), and phenethylammonium. Among them, methylamine, ethylamine, propylamine, butylamine, pentylamine, hexylamine, formamidine and their ions, and phenethylammonium are preferred, and methylamine, ethylamine, propylamine, formamidine and their ions are more preferred.

The M is a metal atom. Examples thereof include lead, tin, zinc, titanium, antimony, bismuth, nickel, iron, cobalt, silver, copper, gallium, germanium, magnesium, calcium, indium, aluminum, manganese, chromium, molybdenum, and europium. These metal atoms may be used alone or may be used in combination of two or more thereof.

The X is a halogen atom or a chalcogen atom. Examples thereof include chlorine, bromine, iodine, sulfur, and selenium. These halogen atoms or chalcogen atoms may be used alone or may be used in combination of two or more thereof. Among them, a halogen atom is preferred because the organic-inorganic perovskite compound containing halogen in the structure is soluble in an organic solvent and is usable in an inexpensive printing method or the like. In addition, iodine is more preferred because the organic-inorganic perovskite compound has a narrow energy band gap.

The organic-inorganic perovskite compound preferably has a cubic structure where the metal atom M is placed at the body center, the organic molecule R is placed at each vertex, and the halogen atom or chalcogen atom X is placed at each face center.

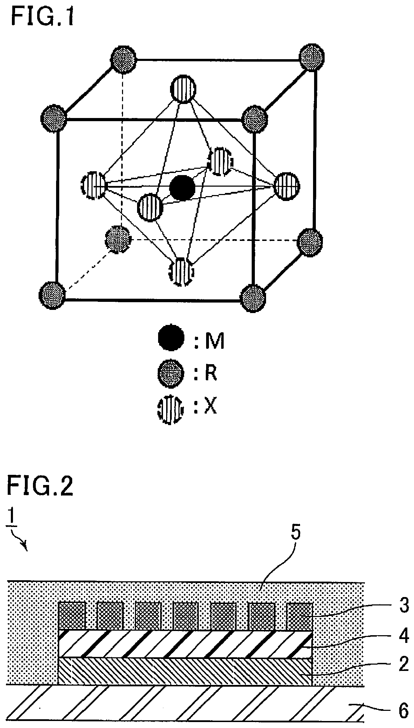

Fig. 1 is a schematic view illustrating an exemplary crystal structure of the organic-inorganic perovskite compound having a cubic structure where the metal atom M is placed at the body center, the organic molecule R is placed at each vertex, and the halogen atom or chalcogen atom X is placed at each face center. Although details are not clear, it is presumed that the direction of an octahedron in the crystal lattice can be easily changed owing to the structure; thus the mobility of electrons in the organic-inorganic perovskite compound is enhanced, improving the photoelectric conversion efficiency of the solar cell.

The organic-inorganic perovskite compound is preferably a crystalline semiconductor. The crystalline semiconductor means a semiconductor whose scattering peak can be detected by the measurement of X-ray scattering intensity distribution. When the organic-inorganic perovskite compound is a crystalline semiconductor, the mobility of electrons in the organic-inorganic perovskite compound is enhanced, improving the photoelectric conversion efficiency of the solar cell.

The degree of crystallinity can also be evaluated as an index of crystallization. The degree of crystallinity can be determined by separating a crystalline substance-derived scattering peak from an amorphous portion-derived halo, which are detected by X-ray scattering intensity distribution measurement, by fitting, determining their respective intensity integrals, and calculating the ratio of the crystalline portion to the whole.

The lower limit of the degree of crystallinity of the organic-inorganic perovskite compound is preferably 30%. When the degree of crystallinity is 30% or more, the mobility of electrons in the organic-inorganic perovskite compound is enhanced, improving the photoelectric conversion efficiency of the solar cell. The lower limit of the degree of crystallinity is more preferably 50%, further preferably 70%.

Examples of the method for increasing the degree of crystallinity of the organic-inorganic perovskite compound include heat annealing, irradiation with light having strong intensity, such as laser, and plasma irradiation.

The photoelectric conversion layer may further include an organic semiconductor or an inorganic semiconductor, in addition to the organic-inorganic perovskite compound, without impairing the effects of the present invention. In this context, the organic semiconductor or the inorganic semiconductor may play a role as an electron transport layer or a hole transport layer mentioned later.

Examples of the organic semiconductor include compounds having a thiophene skeleton, such as poly(3-alkylthiophene). Examples thereof also include conductive polymers having a poly-p-phenylenevinylene skeleton, a polyvinylcarbazole skeleton, a polyaniline skeleton, a polyacetylene skeleton or the like. Examples thereof further include: compounds having a phthalocyanine skeleton, a naphthalocyanine skeleton, a pentacene skeleton, a porphyrin skeleton such as a benzoporphyrin skeleton, a spirobifluorene skeleton or the like; and carbon-containing materials such as carbon nanotube, graphene, and fullerene, which may be surface-modified.

Examples of the inorganic semiconductor include titanium oxide, zinc oxide, indium oxide, tin oxide, gallium oxide, tin sulfide, indium sulfide, zinc sulfide, $CuSCN$, $CU_2O$, $CuI$, $MoO_3$, $V_2O_5$, $WO_3$, $MoS_2$, $MoSe_2$ and $Cu_2S$.

The photoelectric conversion layer including the organic semiconductor or the inorganic semiconductor may be a laminated structure where a thin film-shaped organic semiconductor or inorganic semiconductor part and a thin film-shaped organic-inorganic perovskite compound part are laminated, or may be a composite structure where an organic semiconductor or inorganic semiconductor part and an organic-inorganic perovskite compound part are combined. The laminated structure is preferred from the viewpoint that the production process is simple. The composite structure is preferred from the viewpoint that the charge separation efficiency of the organic semiconductor or the inorganic semiconductor can be improved.

The lower limit of the thickness of the thin film-shaped organic-inorganic perovskite compound part is preferably 5 nm, and the upper limit thereof is preferably 5,000 nm. When the thickness is 5 nm or larger, light can be sufficiently absorbed, enhancing the photoelectric conversion efficiency. When the thickness is 5,000 nm or smaller, presence of a region in which charge separation cannot be achieved can be avoided, leading to higher photoelectric conversion efficiency. The lower limit of the thickness is more preferably 10 nm, and the upper limit thereof is more preferably 1,000 nm. The lower limit of the thickness is further preferably 20 nm, and the upper limit thereof is further preferably 500 nm.

When the photoelectric conversion layer is a composite structure where an organic semiconductor or inorganic semiconductor part and an organic-inorganic perovskite compound part are combined, the lower limit of the thickness of the composite structure is preferably 30 nm, and the upper limit thereof is preferably 3,000 nm. When the thickness is 30 nm or larger, light can be sufficiently absorbed, enhancing the photoelectric conversion efficiency. When the thickness is 3,000 nm or smaller, charge easily arrives at the electrode, enhancing the photoelectric conversion efficiency. The lower limit of the thickness is more preferably 40 nm, and the upper limit thereof is more preferably 2,000 nm. The lower limit of the thickness is further preferably 50 nm, and the upper limit thereof is further preferably 1,000 nm.

In the laminate, an electron transport layer may be disposed between the electrode and the photoelectric conversion layer.

Examples of the material for the electron transport layer include, but are not particularly limited to, N-type conductive polymers, N-type low-molecular organic semiconductors, N-type metal oxides, N-type metal sulfides, alkali metal halides, alkali metals, and surfactants. Specific examples thereof include cyano group-containing polyphenylenevinylene, boron-containing polymers, bathocuproine, bathophenanthroline, hydroxyquinolinatoaluminum, oxadiazole compounds, benzimidazole compounds, naphthalenetetracarboxylic acid compounds, perylene derivatives, phosphine oxide compounds, phosphine sulfide compounds, fluoro group-containing phthalocyanine, titanium oxide, zinc oxide, indium oxide, tin oxide, gallium oxide, tin sulfide, indium sulfide, and zinc sulfide.

The electron transport layer may consist only of a thin film-shaped electron transport layer and preferably includes a porous electron transport layer. Particularly, when the photoelectric conversion layer is a composite structure where an organic semiconductor or inorganic semiconductor part and an organic-inorganic perovskite compound part are combined, a film of the composite structure is preferably formed on a porous electron transport layer because a more complicated composite structure (more intricate structure) is obtained, enhancing the photoelectric conversion efficiency.

The lower limit of the thickness of the electron transport layer is preferably 1 nm, and the upper limit thereof is preferably 2,000 nm. When the thickness is 1 nm or larger, holes can be sufficiently blocked. When the thickness is 2,000 nm or smaller, the layer is less likely to be the resistance to the electron transport, enhancing the photoelectric conversion efficiency. The lower limit of the thickness of the electron transport layer is more preferably 3 nm, and the upper limit thereof is more preferably 1,000 nm. The lower limit of the thickness is further preferably 5 nm, and the upper limit thereof is further preferably 500 nm.

In the laminate, a hole transport layer may be disposed between the counter electrode and the photoelectric conversion layer.

Examples of the material of the hole transport layer include, but are not particularly limited to, P-type conductive polymers, P-type low-molecular organic semiconductors, P-type metal oxides, P-type metal sulfides, and surfactants. Specific examples thereof include polystyrenesulfonic acid adducts of polyethylenedioxythiophene, carboxyl group-containing polythiophene, phthalocyanine, porphyrin, molybdenum oxide, vanadium oxide, tungsten oxide, nickel oxide, copper oxide, tin oxide, molybdenum sulfide, tungsten sulfide, copper sulfide, tin sulfide, fluoro group-containing phosphonic acid, carbonyl group-containing phosphonic acid, copper compounds such as CuSCN and CuI, and carbon-containing materials such as carbon nanotube and graphene, which may be surface-modified.

The lower limit of the thickness of the hole transport layer is preferably 1 nm, and the upper limit thereof is preferably 2,000 nm. When the thickness is 1 nm or larger, electrons can be sufficiently blocked. When the thickness is 2,000 nm or smaller, the layer is less likely to be the resistance to the hole transport, enhancing the photoelectric conversion efficiency. The lower limit of the thickness is more preferably 3 nm, and the upper limit thereof is more preferably 1,000 nm. The lower limit of the thickness is further preferably 5 nm, and the upper limit thereof is further preferably 500 nm.

The laminate may further have a substrate or the like. Examples of the substrate include, but are not particularly limited to, transparent glass substrates such as soda lime glass and alkali-free glass substrates, ceramic substrates and transparent plastic substrates.

In the solar cell of the present invention, the laminate is encapsulated with an encapsulation material. Encapsulation of the laminate with the encapsulation material can improve the durability of the solar cell. This is probably because encapsulation with the encapsulation material can suppress moisture penetration into the inside. In this context, the encapsulation material preferably covers the laminate entirely so as to close the end portions thereof. This can reliably prevent moisture penetration into the inside.

Either electrode side or counter electrode side of the laminate may be covered with an encapsulation material as long as the laminate is encapsulated with the encapsulation material.

The encapsulation material includes a (meth)acrylic resin having a C atom/O atom ratio of 4 or more in the molecule (hereinafter, also simply referred to as a "(meth)acrylic resin").

When the organic-inorganic perovskite compound is used in the photoelectric conversion layer, during encapsulation, an organic component in the organic-inorganic perovskite compound is dissolved into the encapsulation material so that the organic-inorganic perovskite compound is degraded (initial degradation). By contrast, in the solar cell of the present invention, use of the (meth)acrylic resin can prevent elution of an organic component in the organic-inorganic perovskite compound during encapsulation and thus prevent degradation of the photoelectric conversion layer, even when the organic-inorganic perovskite compound is used in the photoelectric conversion layer. This is probably because the (meth)acrylic resin has relatively high hydrophobicity and low affinity for the organic-inorganic perovskite compound. In addition, use of the (meth)acrylic resin in the encapsulation material can suppress time-dependent molecular diffusion and can therefore improve the heat-resistant durability of the solar cell.

The (meth)acrylic resin has a C atom/O atom ratio of preferably 5 or more, more preferably 6 or more, in the molecule. Also, the (meth)acrylic resin has a C atom/O atom ratio of preferably 30 or less, more preferably 20 or less, in the molecule from the viewpoint of the solvent solubility of the resin.

The value of the C atom/O atom ratio in the molecule of the (meth)acrylic resin can be measured by, for example, CHN/O elemental analysis using an organic trace element analyzer (e.g., 2400 II available from PerkinElmer Inc.) or solution NMR using a NMR apparatus (e.g., ECA II available from JEOL Ltd.).

The value of the C atom/O atom ratio in the molecule of the (meth)acrylic polymer can be easily controlled by adjusting the type and composition of a (meth)acrylic monomer used as a raw material.

Specifically, the (meth)acrylic polymer can be obtained, for example, by the homopolymerization or copolymerization of a (meth)acrylic monomer having a C atom/O atom ratio of 4 or more in the molecule.

Examples of the (meth)acrylic monomer having a C atom/O atom ratio of 4 or more in the molecule include: alkyl (meth)acrylates having an alkyl group with 8 or more carbon atoms, such as ethylhexyl (meth)acrylate, lauryl (meth)acrylate, and stearyl (meth)acrylate; aromatic skeleton-containing (meth)acrylates such as phenyl (meth)acrylate and naphthyl (meth)acrylate; alicyclic skeleton-containing (meth)acrylates such as isobornyl (meth)acrylate, norbornyl (meth)acrylate, adamantyl (meth)acrylate, and cyclohexyl (meth)acrylate; and (meth)acrylates having a group to which a reactive functional group can be added (e.g., a hydroxy group, a carboxyl group or an epoxy group), such as hydroxylethylhexyl (meth)acrylate. These (meth)acrylic monomers may be used alone or may be used in combination of two or more thereof. Among them, alkyl (meth)acrylates having an alkyl group with 8 or more carbon atoms, alicyclic skeleton-containing (meth)acrylates, (meth)acrylates having a group to which a reactive functional group can be added (e.g., a hydroxy group, a carboxyl group, or an epoxy group), and the like are preferred, and alicyclic skeleton-containing (meth)acrylates are more preferred.

A (meth)acrylate having a group to which a reactive functional group can be added (e.g., a hydroxy group, a carboxyl group, or an epoxy group) is used as the raw material (meth)acrylic monomer, and the number of the groups to which a reactive functional group can be added is adjusted to control the number of the reactive functional groups to be added. This decreases the cure shrinkage of the encapsulation material to suppress peeling thereof from a finely patterned electrode, and can improve even the high-temperature durability of the solar cell.

Furthermore, adhesion of the encapsulation material to an adherend over a wide temperature range is easily controlled by adjusting the type and composition of the raw material (meth)acrylic monomer. Therefore, even the temperature cycle resistance of the solar cell can be improved.

In consideration of outdoor use, the solar cell is required to be resistant to even an inhospitable environment. Therefore, the encapsulation material may be covered with an inorganic layer as mentioned later.

In this case, use of the (meth)acrylic resin obtained by homopolymerization or copolymerization of a monomer including the alicyclic skeleton-containing (meth)acrylate allows the encapsulation material to be also excellent in sputtering resistance required for forming an inorganic layer by a sputtering method, as compared with encapsulation materials including other resins such as a polyisobutylene resin.

Alternatively, the (meth)acrylic resin may be a resin obtained by forming a film of a copolymer having a reactive functional group, followed by a cross-linking reaction of the reactive functional group using a cross-linking agent. In this case, the number of the reactive functional groups is adjusted to thereby suppress the degradation of the solar cell during encapsulation (initial degradation) caused by cure shrinkage associated with the cross-linking reaction and to improve the sputtering resistance. Examples of the reactive functional group include an epoxy group, a hydroxy group, a carboxyl group, an alkenyl group and an isocyanate group.

The cross-linking agent is not particularly limited, and the cross-linking reaction of the reactive functional group can be initiated using a catalyst or the like.

Alternatively, the (meth)acrylic resin may be a resin obtained by forming a film of the (meth)acrylic monomer in a monomer state, followed by the cross-linking or polymerization of the (meth)acrylic monomer using heat, UV, or the like.

Specific examples of the (meth)acrylic resin include a 2-methacryloyloxyethyl isocyanate adduct (having a methacryloyloxy group as the reactive functional group) of a copolymer of isobornyl acrylate, ethylhexyl acrylate, and hydroxybutyl acrylate ((meth)acrylate having a hydroxy group as the group to which a reactive functional group can be added), and a 2-methacryloyloxyethyl isocyanate adduct (having a methacryloyloxy group as the reactive functional group) of a copolymer of isobornyl acrylate, ethylhexyl acrylate, and acryloyloxyethyl-succinic acid ((meth)acrylate having a carboxyl group as the group to which a reactive functional group can be added).

The lower limit of the solubility parameter (SP value) of the (meth)acrylic resin is preferably 7.0, and the upper limit thereof is preferably 10.0. When the solubility parameter (SP value) of the (meth)acrylic resin is 7.0 or higher, more resins are selectable and such a resin is easier to mold. When the solubility parameter (SP value) of the (meth)acrylic resin is 10.0 or lower, during encapsulation, elution of an organic component in the organic-inorganic perovskite compound can be further prevented and degradation of the photoelectric conversion layer can be further suppressed. The lower limit of the solubility parameter (SP value) of the (meth)acrylic resin is more preferably 7.5, further preferably 8.0. The upper limit of the solubility parameter (SP value) of the encapsulation resin is more preferably 9.5, further preferably 9.0, from the viewpoint of enhancing the high-temperature durability of the solar cell.

The SP value is called the solubility parameter and is an index capable of showing ease of dissolution. The SP value herein can be determined by a method proposed by Fedors (R.F. Fedors, Polym. Eng. Sci., 14 (2), 147-154 (1974)), and calculated according to the equation (1) given below based on the evaporation energy ($\Delta$ecoh) (cal/mol) and molar volume ($\Delta$v) (cm$^3$/mol) of each atomic group in repeating units. In the equation (1), $\delta$ represents the SP value (cal/mol)$^{1/2}$.

$$\delta = \sqrt{\frac{\sum \Delta ecoh}{\sum \Delta v}} \qquad (1)$$

Values described in J. Brandrup et al., "Polymer Handbook, Fourth Edition", volume 2 can be used as $\Delta$ecoh and $\Delta$v.

In the case of Tg $\geq$ 25°C, 2n (n represents the number of main chain atoms) at n $\geq$ 3 or 4n at n < 3 is added to $\Delta$v for the calculation.

The SP value of the copolymer can be calculated according to the equation (2) given below using the calculated SP value of each repeating unit alone in the copolymer, and the volume fraction thereof. In the equation (2), 8cop represents the SP value of the copolymer, $\phi$1 and $\phi$2 represent the respective volume fractions of repeating units 1 and 2, and $\delta$1 and $\delta$2 represent the respective SP values of repeating units 1 and 2 each calculated alone.

$$\delta cop^2 = \emptyset_1 \delta_1^2 + \emptyset_2 \delta_2^2 \qquad (2)$$

The lower limit of the thickness of the encapsulation material is preferably 100 nm, and the upper limit thereof is preferably 100,000 nm. The lower limit of the thickness is more preferably 500 nm, and the upper limit thereof is more preferably 50,000 nm. The lower limit of the thickness is further preferably 1,000 nm, and the upper limit thereof is further preferably 20,000 nm.

Preferably, the solar cell of the present invention further includes an inorganic layer on the encapsulation material. Having a water vapor barrier property, the inorganic layer can suppress moisture penetration into the inside and can therefore improve the high-humidity durability of the solar cell.

The inorganic layer preferably contains a metal oxide, a metal nitride, or a metal oxynitride.

The metal oxide, metal nitride, or metal oxynitride is not particularly limited as long as it has a water vapor barrier property. Examples thereof include an oxide, nitride, or oxynitride of Si, Al, Zn, Sn, In, Ti, Mg, Zr, Ni, Ta, W, Cu, or an alloy containing two or more of them. Among them, an oxide, nitride, or oxynitride of Si, Al, Zn, or Sn is preferred, and an oxide, nitride, or oxynitride of Zn or Sn is more preferred. An oxide, a nitride, or an oxynitride of metal elements including both of the metal elements Zn and Sn is further preferred because a particularly high water vapor barrier property and plasticity can be imparted to the inorganic layer.

Among others, the metal oxide, metal nitride, or metal oxynitride is particularly preferably a metal oxide represented by the formula $Zn_aSn_bO_c$. In this formula, a, b and c each represent a positive integer.

Use of the metal oxide represented by the formula $Zn_aSn_bO_c$ in the inorganic layer can impart moderate flexibility to the inorganic layer because the metal oxide contains a tin (Sn) atom, so that stress is decreased even when the thickness of the inorganic layer is increased. Therefore, peeling of the inorganic layer, electrode, semiconductor layer, and the like can be suppressed. This can enhance the water vapor barrier property of the inorganic layer and further improve the durability of the solar cell. Meanwhile, the inorganic layer can exert a particularly high barrier property because the metal oxide contains a zinc (Zn) atom.

In the metal oxide represented by the formula $Zn_aSn_bO_c$, the ratio Xs (% by weight) of Sn to the total sum of Zn and Sn preferably satisfies 70 > Xs > 0. Also, value Y represented by Y = c / (a + 2b) preferably satisfies 1.5 > Y > 0.5.

The element ratios of zinc (Zn), tin (Sn), and oxygen (O) contained in the metal oxide represented by the formula $Zn_aSn_bO_c$ in the inorganic layer can be measured using an X-ray photoemission spectroscopy (XPS) surface analyzer (e.g., ESCALAB-200R available from VG Scientific).

Preferably, the inorganic layer containing the metal oxide represented by the formula $Zn_aSn_bO_c$ further contains silicon (Si) and/or aluminum (Al).

The addition of silicon (Si) and/or aluminum (Al) to the inorganic layer can enhance the transparency of the inorganic layer and improve the photoelectric conversion efficiency of the solar cell.

The lower limit of the thickness of the inorganic layer is preferably 30 nm, and the upper limit thereof is preferably 3,000 nm. When the thickness is 30 nm or larger, the inorganic layer can have an adequate water vapor barrier property, improving the durability of the solar cell. When the thickness is 3,000 nm or smaller, only small stress is generated even when the thickness of the inorganic layer is increased. Therefore, peeling of the inorganic layer, electrode, semiconductor layer, and the like can be suppressed. The lower limit of the thickness is more preferably 50 nm, and the upper limit thereof is more preferably 1,000 nm. The lower limit of the thickness is further preferably 100 nm, and the upper limit thereof is further preferably 500 nm.

The thickness of the inorganic layer can be measured using an optical interference-type film thickness measurement apparatus (e.g., FE-3000 available from Otsuka Electronics Co., Ltd.).

In the solar cell of the present invention, the encapsulation material may be further covered with, for example, an additional material such as a glass sheet, resin film, inorganic material-coated resin film, or metal (e.g., aluminum) foil. Specifically, the solar cell of the present invention may be configured such that encapsulation, filling, or bonding between the laminate and the additional material is attained by the encapsulation material. This can sufficiently block water vapor even when a pinhole is present in the encapsulation material, and can further improve the high-humidity durability of the solar cell. Among them, an inorganic material-coated resin film is more preferably disposed thereon.

Fig. 2 is a cross-sectional view schematically illustrating an exemplary solar cell of the present invention.

In a solar cell 1 shown in Fig. 2, a laminate having, on a substrate 6, an electrode 2, a counter electrode 3, and a photoelectric conversion layer 4 disposed between the electrode 2 and the counter electrode 3 is encapsulated with an encapsulation material 5 that covers the counter electrode 3. In this context, the end portions of the encapsulation material 5 are closed by intimate contact with the substrate 6. In the solar cell 1 shown in Fig. 2, the counter electrode 3 is a patterned electrode. An inorganic layer (not shown) may be disposed between the laminate and the encapsulation material 5 or on the encapsulation material 5.

Examples of the method for producing the solar cell of the present invention include, but are not particularly limited to, a method which involves forming the electrode, the photoelectric conversion layer, and the counter electrode in this order on the substrate to prepare a laminate, then encapsulating the laminate with the encapsulation material, and further covering the encapsulation material with an inorganic layer.

Examples of the method for forming the photoelectric conversion layer include, but are not particularly limited to, a vapor deposition method, a sputtering method, a chemical vapor deposition (CVD) method, an electrochemical deposition method, and a printing method. Among them, employment of a printing method allows simple formation of a large-area solar cell that can exhibit high photoelectric conversion efficiency. Examples of the printing method include a spin coating method and a casting method. Examples of the method using the printing method include a roll-to-roll method.

Examples of the method for encapsulating the laminate with the encapsulation material include, but are not particularly limited to, a method which involves sealing the laminate using a sheet-shaped encapsulation material, a method which involves applying an encapsulation material solution containing the encapsulation material dissolved in an organic solvent to the laminate, a method which involves applying a compound having a reactive functional group to be the encapsulation material to the laminate, followed by cross-linking or polymerization of the compound having a reactive functional group using heat, UV, or the like, and a method which involves melting the encapsulation material under heat, followed by cooling.

The method for covering the encapsulation material with the inorganic layer is preferably a vacuum deposition method, a sputtering method, a chemical vapor deposition (CVD) method, or an ion plating method. Among them, a sputtering method is preferred for forming a dense layer. The sputtering method is more preferably a DC magnetron sputtering method. Use of the (meth)acrylic resin prepared from the alicyclic skeleton-containing (meth)acrylate as a raw material allows the encapsulation material to be also excellent in sputtering resistance required for forming the inorganic layer by the sputtering method, as compared with encapsulation materials including other resins such as a polyisobutylene resin. When the (meth)acrylic resin is a resin obtained by forming a film of a copolymer having a reactive functional group, followed by a cross-linking reaction of the reactive functional group using a cross-linking agent, the sputtering resistance can also be improved.

In the sputtering method, the inorganic layer can be formed by depositing raw materials including a metal target and oxygen gas or nitrogen gas on the encapsulation material for film formation.

- Advantageous Effects of Invention

The present invention can provide a solar cell that is excellent in photoelectric conversion efficiency, suffers little degradation during encapsulation (initial degradation), has high-temperature durability, and is excellent in temperature cycle resistance.

BRIEF DESCRIPTION OF DRAWINGS

Fig. 1 is a schematic view illustrating an exemplary crystal structure of the organic-inorganic perovskite compound.
Fig. 2 is a cross-sectional view schematically illustrating an exemplary solar cell of the present invention.

DESCRIPTION OF EMBODIMENTS

Hereinafter, the present invention will be described in more detail with reference to Examples. However, the present invention is not intended to be limited by these Examples.

(Example 1)

(Preparation of laminate)

A FTO film having a thickness of 1,000 nm was formed as an electrode on a glass substrate, ultrasonically washed with pure water, acetone, and methanol each for ten minutes in the stated order, and then dried.
An ethanol solution of titanium isopropoxide adjusted to 2% was applied onto the surface of the FTO film by the spin coating method and then fired at 400°C for 10 minutes to form a thin film-shaped electron transport layer having a thickness of 20 nm. A titanium oxide paste containing polyisobutyl methacrylate as an organic binder and titanium oxide (mixture of powders having average particle sizes of 10 nm and 30 nm) was further applied onto the thin film-shaped electron transport layer by the spin coating method and then fired at 500°C for 10 minutes to form a porous electron transport layer having a thickness of 500 nm.
Subsequently, $CH_3NH_3I$ and $PbI_2$ were dissolved at a molar ratio of 1:1 in N,N-dimethylformamide (DMF) as a solvent to prepare a solution for organic-inorganic perovskite compound formation having a total concentration of $CH_3NH_3I$ and $PbI_2$ of 20% by weight. This solution was laminated onto the electron transport layer by the spin coating method to form a photoelectric conversion layer.
Further, 68 mM spiro-OMeTAD (having a spirobifluorene skeleton), 55 mM tert-butylpyridine and 9 mM lithium bis(trifluoromethylsufonyl)imide salt were dissolved in 25 $\mu$L of chlorobenzene to prepare a solution. This solution was laminated to a thickness of 300 nm onto the photoelectric conversion layer by the spin coating method to form a hole transport layer.
A gold film having a thickness of 100 nm was formed as a counter electrode on the hole transport layer by vacuum deposition to obtain a laminate.

(Encapsulation of laminate)

A mixture containing a 2-methacryloyloxyethyl isocyanate (MOI, available from Showa Denko K.K.) adduct (having a methacryloyloxy group as the reactive functional group) of a copolymer of isobornyl acrylate (iB, available from Kyoeisha Chemical Co., Ltd.), ethylhexyl acrylate (EH, available from Mitsubishi Chemical Corp.), and acryloyloxyethyl-succinic acid ((meth)acrylate having a carboxyl group as the group to which a reactive functional group can be added; available from Kyoeisha Chemical Co., Ltd.), and a reaction catalyst peroxide (Percumyl D, available from NOF Corp.) was laminated to a thickness of 10 $\mu$m on the obtained laminate using a doctor blade, followed by a cross-linking reaction of the copolymer at 150°C for 10 minutes to prepare an encapsulation material.
The added monomer ratio of iB, EH and MOI was 4.5:4.5:1 (molar ratio). As a result of measurement by CHN/O elemental analysis, the C atom/O atom ratio in the molecule of the obtained copolymer was 6.

(Formation of inorganic layer)

The obtained laminate was set in a substrate holder of a sputtering device. Further, a ZnSn alloy (Zn:Sn = 95:5% by weight) target was mounted on Cathode A of the sputtering device, and a Si target was mounted on Cathode B of the sputtering device. A film forming chamber of the sputtering device was evacuated using a vacuum pump to reduce the pressure to $5.0 \times 10^{-4}$ Pa. Then, sputtering was performed under the condition shown as Sputtering condition A to form a 100 nm ZnSnO(Si) thin film as an inorganic film (encapsulation layer) on the laminate. A thin-film solar cell was thus obtained.

<Sputtering conditions A>

Argon gas flow rate: 50 sccm, oxygen gas flow rate: 50 sccm

Power output: Cathode A = 500 W, Cathode B = 1500 W

(Examples 2 to 5)

A solar cell was obtained in the same manner as in Example 1, except that in preparation of the laminate, the components contained in the solution for organic-inorganic perovskite compound formation was changed to form a photoelectric conversion layer (organic-inorganic perovskite compound) shown in Table 1.

In Example 2, $CH_3NH_3Br$, $CH_3NH_3I$, $PbBr_2$, and $PbI_2$ were dissolved at a molar ratio of 1:2:1:2 in N,N-dimethylformamide (DMF) as a solvent. In Example 3, $CH_3NH_3I$ and $PbCl_2$ were dissolved at a molar ratio of 3:1 in N,N-dimethylformamide (DMF) as a solvent. In Example 4, $CH_3NH_3Br$ and $PbBr_2$ were dissolved at a molar ratio of 1:1 in N,N-dimethylformamide (DMF) as a solvent. In Example 5, $CH_3(NH_3)_2I$ and $PbI_2$ were dissolved at a molar ratio of 1:1 in N,N-dimethylformamide (DMF) as a solvent.

(Example 6)

A solar cell was obtained in the same manner as in Example 1, except that in encapsulation of the laminate, the encapsulation material thickness was changed as shown in Table 1.

(Examples 7 to 9)

A solar cell was obtained in the same manner as in Example 1, except that in encapsulation of the laminate, the encapsulation material was changed to that shown in Table 1.

In Example 7, a copolymer of isobornyl acrylate (iB) and ethylhexyl acrylate (EH) was used. The C atom/O atom ratio in the molecule of the obtained copolymer was 6. The added monomer ratio of iB and EH was 5:5 (molar ratio).

In Example 8, a 2-methacryloyloxyethyl isocyanate (MOI) adduct (having a methacryloyloxy group as the reactive functional group) of a copolymer of isobornyl acrylate (iB) and acryloyloxyethyl-succinic acid ((meth)acrylate having a carboxyl group as the group to which a reactive functional group can be added) was used. The C atom/O atom ratio in the molecule of the obtained copolymer was 6.5. The added monomer ratio of iB and MOI was 9:1 (molar ratio).

In Example 9, a 2-methacryloyloxyethyl isocyanate (MOI) adduct (having a methacryloyloxy group as the reactive functional group) of a copolymer of ethylhexyl acrylate (EH) and acryloyloxyethyl-succinic acid ((meth)acrylate having a carboxyl group as the group to which a reactive functional group can be added) was used. The C atom/O atom ratio in the molecule of the obtained copolymer was 5.5. The added monomer ratio of EH and MOI was 9:1 (molar ratio).

(Examples 10 to 12)

A solar cell was obtained in the same manner as in Example 1 except that: the encapsulation material was laminated after formation of the inorganic layer on the laminate, instead of forming the inorganic layer on the encapsulation material; and the inorganic layer was changed as specified in Table 1.

In Example 11, a Si target was used as a metal target. In Example 12, a Sn target was used as a metal target.

(Example 13)

A solar cell was obtained in the same manner as in Example 1, except that the inorganic layer was not formed on the encapsulation material.

(Examples 14 to 16)

A solar cell was obtained in the same manner as in Example 1, except that in encapsulation of the laminate, the encapsulation material was changed to that shown in Table 1.

In Example 14, a 2-methacryloyloxyethyl isocyanate (MOI) adduct (having a methacryloyloxy group as the reactive functional group) of a copolymer of cyclohexyl acrylate (CH, available from Tokyo Chemical Industry Co., Ltd.) and acryloyloxyethyl-succinic acid ((meth)acrylate having a carboxyl group as the group to which a reactive functional group can be added) was used. The C atom/O atom ratio in the molecule of the obtained copolymer was 4.5. The added monomer ratio of CH and MOI was 9:1 (molar ratio).

In Example 15, a 2-methacryloyloxyethyl isocyanate (MOI) adduct (having a methacryloyloxy group as the reactive functional group) of a copolymer of t-butyl methacrylate (tB, available from Tokyo Chemical Industry Co., Ltd.) and acryloyloxyethyl-succinic acid ((meth)acrylate having a carboxyl group as the group to which a reactive functional group can be added) was used. The C atom/O atom ratio in the molecule of the obtained copolymer was 4. The added monomer ratio of tB and MOI was 9:1 (molar ratio).

In Example 16, methyl acrylate (Me, available from Mitsubishi Chemical Corp.) was used instead of ethylhexyl acrylate (EH, available from Mitsubishi Chemical Corp.). The C atom/O atom ratio in the molecule of the obtained copolymer was 4.5.

(Comparative Examples 1 to 5)

A solar cell was obtained in the same manner as in Example 1, except that in encapsulation of the laminate, the encapsulation material was changed to that shown in Table 1.

In Comparative Example 1, a solution of polyvinyl alcohol (PVA) (available from Wako Pure Chemical Industries, Ltd.) was applied onto the laminate using a doctor blade and dried to prepare an encapsulation material.

In Comparative Example 2, a mixture containing 4 mol% of an imidazole compound 2MZA (available from Shikoku Chemicals Corp.) as a curing agent and a bisphenol A epoxy resin (available from Mitsubishi Chemical Corp.) was applied onto the laminate and cured by heating at 120°C for one hour to prepare an encapsulation material.

In Comparative Example 3, a solution of a polyisobutylene resin (OPPANOL B 50, available from BASF SE) was applied onto the laminate using a doctor blade and dried to prepare an encapsulation material.

In Comparative Example 4, a solution of a norbornene resin (available from Polyplastics Co., Ltd.) was applied onto the laminate using a doctor blade and dried to prepare an encapsulation material. In Comparative Example 5, a solution of a polymethyl methacrylate resin (available from Wako Pure Chemical Industries, Ltd.) was applied onto the laminate using a doctor blade and dried to prepare an encapsulation material.

In Comparative Example 5, a 2-methacryloyloxyethyl isocyanate (MOI) adduct (having a methacryloyloxy group as the reactive functional group) of a copolymer of t-butyl acrylate (tB, available from Osaka Organic Chemical Industry Ltd.) and acryloyloxyethyl-succinic acid ((meth)acrylate having a carboxyl group as the group to which a reactive functional group can be added) was used. The C atom/O atom ratio in the molecule of the obtained copolymer was 3.5. The added monomer ratio of tB and MOI was 9:1 (molar ratio).

(Comparative Example 6)

A solar cell was obtained in the same manner as in Example 1, except that encapsulation of the laminate was not performed.

<Evaluation>

The solar cells obtained in Examples and Comparative Examples were evaluated as described below.

(1) Degradation during encapsulation (initial degradation)

A power source (236 model, available from Keithley Instruments, Inc.) was connected between the electrodes in the laminate before encapsulation. The photoelectric conversion efficiency was measured using a solar simulator (available from Yamashita Denso Corp.) having an intensity of 100 mW/cm$^2$, and the obtained value was taken as the initial conversion efficiency.

A power source (236 model, available from Keithley Instruments, Inc.) was connected between the electrodes in the solar cell immediately after encapsulation. The photoelectric conversion efficiency was measured using a solar simulator (available from Yamashita Denso Corp.) having an intensity of 100 mW/cm$^2$ to determine the value of photoelectric conversion efficiency immediately after encapsulation/initial conversion efficiency.

○ (Good): The value of photoelectric conversion efficiency immediately after encapsulation/initial conversion efficiency was 0.5 or more.

✕ (Poor): The value of photoelectric conversion efficiency immediately after encapsulation/initial conversion efficiency was less than 0.5.

(2) High-humidity durability

The solar cell was left for 24 hours under conditions of 70% and 30°C to conduct a high-humidity durability test. A power source (236 model, available from Keithley Instruments, Inc.) was connected between the electrodes in the solar cell after the high-humidity durability test. The photoelectric conversion efficiency was measured using a solar simulator (available from Yamashita Denso Corp.) having an intensity of 100 mW/cm$^2$, and the value of photoelectric conversion efficiency after the high-humidity durability test/photoelectric conversion efficiency immediately after encapsulation was determined.

○○ (Excellent): The value of photoelectric conversion efficiency after the high-humidity durability test/photoelectric conversion efficiency immediately after encapsulation was 0.9 or more.
○ (Good): The value of photoelectric conversion efficiency after the high-humidity durability test/photoelectric conversion efficiency immediately after encapsulation was 0.5 or more and less than 0.9.
× (Poor): The value of photoelectric conversion efficiency after the high-humidity durability test/photoelectric conversion efficiency immediately after encapsulation was less than 0.5.

(3) High-temperature durability

The solar cell was heated for 30 minutes on a hot plate set to 150°C to conduct a high-temperature durability test. A power source (236 model, available from Keithley Instruments, Inc.) was connected between the electrodes in the solar cell after the high-temperature durability test. The photoelectric conversion efficiency was measured using a solar simulator (available from Yamashita Denso Corp.) having an intensity of 100 mW/cm$^2$ to determine the value of photoelectric conversion efficiency after the high-temperature durability test/photoelectric conversion efficiency immediately after encapsulation.

○○ (Excellent): The value of photoelectric conversion efficiency after the high-temperature durability test/photoelectric conversion efficiency immediately after encapsulation was 0.9 or more.
○ (Good): The value of photoelectric conversion efficiency after the high-temperature durability test/photoelectric conversion efficiency immediately after encapsulation was 0.7 or more and less than 0.9.
Δ (Average): The value of photoelectric conversion efficiency after the high-temperature durability test/photoelectric conversion efficiency immediately after encapsulation was 0.5 or more and less than 0.7.
× (Poor): The value of photoelectric conversion efficiency after the high-temperature durability test/photoelectric conversion efficiency immediately after encapsulation was less than 0.5.

(4) Temperature cycle resistance

In a temperature cycle test, the solar cell was subjected to 300 cycles of temperature cycling from -55°C to 125°C. A power source (236 model, available from Keithley Instruments, Inc.) was connected between the electrodes in the solar cell after the temperature cycle test. The photoelectric conversion efficiency was measured using a solar simulator (available from Yamashita Denso Corp.) having an intensity of 100 mW/cm$^2$ to determine the value of photoelectric conversion efficiency after the temperature cycle test/photoelectric conversion efficiency immediately after encapsulation.

○ (Good): The value of photoelectric conversion efficiency after the temperature cycle test/photoelectric conversion efficiency immediately after encapsulation was 0.5 or more.
× (Poor): The value of photoelectric conversion efficiency after the temperature cycle test/photoelectric conversion efficiency immediately after encapsulation was less than 0.5.

(5) Sputtering resistance

In the production process of the solar cell, the surface of the encapsulation material was visually observed when the inorganic layer was formed on the encapsulation material by the sputtering method.

○ (Good): Not changed.
Δ (Average): Slight whitening was found on the encapsulation material.
×: Whitening was found on the encapsulation material.

[Table 1]

| | Photoelectric conversion layer | Encapsulation material | C atom/O atom ratio of (meth)acrylic resin | Thickness of encapsulation material (μm) | Inorganic layer Material | Inorganic layer Thickness (nm) | Solar cell structure | Evaluation Initial degradation | Evaluation Humidity resistance | Evaluation High-temperature durability | Evaluation Temperature cycle resistance | Evaluation Sputtering resistance |
|---|---|---|---|---|---|---|---|---|---|---|---|---|
| Example 1 | $CH_3NH_3PbI_3$ | IB/EH-MOI adduct | 6 | 10 | ZnSnO(SO) | 100 | Counter electrode/encapsulation material/inorganic layer | O | OO | OO | O | O |
| Example 2 | $CH_3NH_3PbI_2Br$ | IB/EH-MOI adduct | 6 | 10 | ZnSnO(SO) | 100 | Counter electrode/encapsulation material/inorganic layer | O | OO | OO | O | O |
| Example 3 | $CH_3NH_3PbI_2Cl$ | IB/EH-MOI adduct | 6 | 10 | ZnSnO(SO) | 100 | Counter electrode/encapsulation material/inorganic layer | O | OO | OO | O | O |
| Example 4 | $CH_3NH_3PbBr_3$ | IB/EH-MOI adduct | 6 | 10 | ZnSnO(SO) | 100 | Counter electrode/encapsulation material/inorganic layer | O | OO | OO | O | O |
| Example 5 | $CH_3(NH_3)_2PbI_4$ | IB/EH-MOI adduct | 6 | 10 | ZnSnO(SO) | 100 | Counter electrode/encapsulation material/inorganic layer | O | OO | OO | O | O |
| Example 6 | $CH_3NH_3PbI_3$ | IB/EH-MOI adduct | 6 | 5 | ZnSnO(SO) | 100 | Counter electrode/encapsulation material/inorganic layer | O | OO | OO | O | O |
| Example 7 | $CH_3NH_3PbI_3$ | IB/EH | 6 | 10 | ZnSnO(SO) | 100 | Counter electrode/encapsulation material/inorganic layer | O | OO | OO | O | △ |
| Example 8 | $CH_3NH_3PbI_3$ | IB-MOI adduct | 6.5 | 10 | ZnSnO(SO) | 100 | Counter electrode/encapsulation material/inorganic layer | O | OO | OO | O | O |
| Example 9 | $CH_3NH_3PbI_3$ | EH-MOI adduct | 5.5 | 10 | ZnSnO(SO) | 100 | Counter electrode/inorganic layer/encapsulation material | O | OO | OO | O | △ |
| Example 10 | $CH_3NH_3PbI_3$ | IB/EH-MOI adduct | 6 | 10 | ZnSnO(SO) | 100 | Counter electrode/inorganic layer/encapsulation material | O | OO | OO | O | O |
| Example 11 | $CH_3NH_3PbI_3$ | IB/EH-MOI adduct | 6 | 10 | $SiO_2$ | 100 | Counter electrode/inorganic layer/encapsulation material | O | OO | OO | O | O |
| Example 12 | $CH_3NH_3PbI_3$ | IB/EH-MOI adduct | 6 | 10 | $SnO_2$ | 100 | Counter electrode/inorganic layer/encapsulation material | O | OO | OO | O | O |
| Example 13 | $CH_3NH_3PbI_3$ | IB/EH-MOI adduct | 6 | 10 | — | — | Counter electrode/encapsulation material | O | OO | OO | O | — |
| Example 14 | $CH_3NH_3PbI_3$ | CH-MOI adduct | 4.5 | 10 | ZnSnO(SO) | 100 | Counter electrode/encapsulation material/inorganic layer | O | OO | O | O | O |
| Example 15 | $CH_3NH_3PbI_3$ | tBu-MOI adduct | 4 | 10 | ZnSnO(SO) | 100 | Counter electrode/encapsulation material/inorganic layer | O | OO | △ | O | △ |
| Example 16 | $CH_3NH_3PbI_3$ | IB/Me-MOI adduct | 4.5 | 10 | $SiO_2$ | 100 | Counter electrode/inorganic layer/encapsulation material | O | OO | O | O | O |
| Comparative Example 1 | $CH_3NH_3PbI_3$ | PVA | — | 10 | ZnSnO(SO) | 100 | Counter electrode/encapsulation material/inorganic layer | × | — | — | — | — |
| Comparative Example 2 | $CH_3NH_3PbI_3$ | Epoxy resin | — | 10 | ZnSnO(SO) | 100 | Counter electrode/encapsulation material/inorganic layer | × | — | — | — | — |
| Comparative Example 3 | $CH_3NH_3PbI_3$ | Norbornene resin | — | 10 | ZnSnO(SO) | 100 | Counter electrode/encapsulation material/inorganic layer | O | OO | OO | × | O |
| Comparative Example 4 | $CH_3NH_3PbI_3$ | Polymethylmethacrylate | 2.5 | 10 | $SiO_2$ | 100 | Counter electrode/inorganic layer/encapsulation material | O | OO | × | — | × |
| Comparative Example 5 | $CH_3NH_3PbI_3$ | Polybutyl acrylate | 3.5 | 10 | $SiO_2$ | 100 | Counter electrode/encapsulation material/inorganic layer | O | OO | × | — | × |
| Comparative Example 6 | $CH_3NH_3PbI_3$ | Not used | — | Not used | — | — | Counter electrode | — | × | — | × | — |

INDUSTRIAL APPLICABILITY

The present invention can provide a solar cell that is excellent in photoelectric conversion efficiency, suffers little degradation during encapsulation (initial degradation), has high-temperature durability, and is excellent in temperature cycle resistance.

REFERENCE SIGNS LIST

1:     solar cell
2:     electrode
3:     counter electrode (patterned electrode)
4:     photoelectric conversion layer
5:     encapsulation material
6:     substrate

**Claims**

**1.** A solar cell (1) comprising:

a laminate having an electrode (2), a counter electrode (3), and a photoelectric conversion layer (4) disposed between the electrode (2) and the counter electrode (3); and
an encapsulation material (5) covering the counter electrode (3) to encapsulate the laminate,
the photoelectric conversion layer (4) including an organic-inorganic perovskite compound represented by the formula: $R\text{-}M\text{-}X_3$, R representing an organic molecule, M representing a metal atom, X representing a halogen atom or a chalcogen atom,
**characterized in that**
the encapsulation material (5) includes a (meth)acrylic resin having a C atom/O atom ratio of 4 or more in the molecule and is in direct contact with the photoelectric conversion layer (4).

**2.** The solar cell (1) according to claim 1,
wherein the solar cell further includes an inorganic layer on the encapsulation material (5), and
the inorganic layer contains a metal oxide, a metal nitride, or a metal oxynitride.

**Patentansprüche**

**1.** Solarzelle (1), umfassend:

ein Laminat, aufweisend eine Elektrode (2), eine Gegenelektrode (3) und eine photoelektrische Umwandlungsschicht (4), die zwischen der Elektrode (2) und der Gegenelektrode (3) angeordnet ist, und
ein Einkapselungsmaterial (5), das die Gegenelektrode (3) bedeckt, um das Laminat einzukapseln,
wobei die photoelektrische Umwandlungsschicht (4) eine organischanorganische Perowskitverbindung aufweist, die durch die Formel $R\text{-}M\text{-}X_3$ dargestellt ist, wobei R ein organisches Molekül darstellt, M ein Metallatom darstellt und X ein Halogenatom oder ein Chalkogenatom darstellt,
**dadurch gekennzeichnet, dass**
das Einkapselungsmaterial (5) ein (Meth)Acrylharz mit einem C-Atom/O-AtomVerhältnis von 4 oder mehr in dem Molekül enthält und in direktem Kontakt mit der photoelektrischen Umwandlungsschicht (4) ist.

**2.** Solarzelle (1) nach Anspruch 1,
wobei die Solarzelle ferner eine anorganische Schicht auf dem Einkapselungsmaterial (5) aufweist und
die anorganische Schicht ein Metalloxid, ein Metallnitrid oder ein Metalloxynitrid enthält.

**Revendications**

**1.** Cellule photovoltaïque (1) comprenant :

un stratifié présentant une électrode (2), une contre-électrode (3), et une couche de conversion photoélectrique (4) disposée entre l'électrode (2) et la contre-électrode (3) ; et
un matériau d'encapsulation (5) couvrant la contre-électrode (3) pour encapsuler le stratifié,
la couche de conversion photoélectrique (4) incluant un composé de perovskite organique-inorganique représenté par la formule : $R\text{-}M\text{-}X_3$, R représentant une molécule organique, M représentant un atome de métal, X représentant un atome d'halogène ou un atome de chalcogène,
**caractérisée en ce que**
le matériau d'encapsulation (5) comprend une résine (méth)acrylique présentant un rapport atome C/atome O de 4 ou supérieur dans la molécule et est en contact direct avec la couche de conversion photoélectrique (4).

2. Cellule photovoltaïque (1) selon la revendication 1,
dans laquelle la cellule photovoltaïque comprend de plus une couche inorganique sur le matériau d'encapsulation (5), et
la couche inorganique contient un oxyde de métal, un nitrure de métal, ou un oxynitrure de métal.

FIG.1

● : M

◉ : R

◍ : X

FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006344794 A
- EP 2056372 A2
- WO 2014097299 A1

**Non-patent literature cited in the description**

- **REESE et al.** *Adv. Funct. Mater.,* 2010, vol. 20, 3476-3483
- *Proc. of SPIE,* vol. 7416
- **R.F. FEDORS.** *Polym. Eng. Sci.,* 1974, vol. 14 (2), 147-154
- **J. BRANDRUP et al.** Polymer Handbook. vol. 2